Europäisches Patentamt

European Patent Office

Office européen des brevets

(19)

(11) Numéro de publication : **0 393 049 B1**

# (12) FASCICULE DE BREVET EUROPEEN

(45) Date de publication du fascicule du brevet :
**24.02.93 Bulletin 93/08**

(51) Int. Cl.$^5$ : **A61K 9/50**

(21) Numéro de dépôt : **88908243.4**

(22) Date de dépôt : **14.09.88**

(86) Numéro de dépôt international :
**PCT/FR88/00452**

(87) Numéro de publication internationale :
**WO 89/02267 23.03.89 Gazette 89/07**

(54) **LIPOSOMES, LEUR PROCEDE ET APPLICATION COMME VECTEUR DE PRODUITS.**

Le dossier contient des informations techniques présentées postérieurement au dépôt de la demande et ne figurant pas dans le présent fascicule.

(30) Priorité : **15.09.87 FR 8712750**

(43) Date de publication de la demande :
**24.10.90 Bulletin 90/43**

(45) Mention de la délivrance du brevet :
**24.02.93 Bulletin 93/08**

(84) Etats contractants désignés :
**AT BE CH DE GB IT LI LU NL SE**

(56) Documents cités :
**EP-A- 0 162 724**

(56) Documents cités :
**EP-A- 0 277 776**
**Biochemistry, Vol.18,No12,1979, American Chemical Society, M. Minetti: "Interaction of neutral polysaccharides with phosphatidyl-choline multilamellar liposomes. Phase transitions studies by the binding of fluorescein-conjugated dextrans".**

(73) Titulaire : **HAUTON, Jacques**
**12, boulevard Henri-Fabre**
**F-13012 Marseille (FR)**

(72) Inventeur : **HAUTON, Jacques**
**12, boulevard Henri-Fabre**
**F-13012 Marseille (FR)**

(74) Mandataire : **Brycman, Jean et al**
**CABINET ORES 6, Avenue de Messine**
**F-75008 Paris (FR)**

EP 0 393 049 B1

## Description

La présente invention est relative à des liposomes, à leur procédé de préparation, ainsi qu'à l'application desdits liposomes, notamment comme vecteur de produits à action pharmacologique.

Que ce soit en recherche fondamentale, en recherche clinique, en pharmacologie ou en cosmétologie, les liposomes offrent de nombreuses perspectives dont certaines sont susceptibles d'applications industrielles, telle que, notamment, leur utilisation en tant que vecteurs d'enzymes, de médicaments, d'hormones, d'acides nucléiques, etc ...

Deux types de liposomes sont à distinguer :

- les vésicules multilamellaires (MLV), constituées par une superposition concentrique de phases interfaciales en bicouches séparées entre elles par une phase polaire aqueuse,
- les vésicules unilamellaires, constituées par une seule couche externe d'une phase interfaciale en bicouche entourant une phase polaire aqueuse. Le diamètre de ces vésicules dépend de la méthode de préparation employée ; on distingue ainsi les petits liposomes unilamellaires (SUV) d'un diamètre inférieur à $1000.10^{-8}$ cm ou 1000 Å, et les grands liposome unilamellaires (LUV) d'un diamètre supérieur à $5000.10^{-8}$cm ou 5000 Å.

Les liposomes (ou vésicules unilamellaires ou multilamellaires en bicouche encapsulant une phase polaire aqueuse) peuvent être formés à partir d'une variété de lipides ou mélanges de lipides de la classe 4 [tels que phospholipides] associés ou non à des lipides de la classe 3 [tels que cholestérol libre] ou 2.

Les paramètres physico-chimiques des liposomes peuvent être déterminés par une nouvelle méthode de calcul dérivant du concept biodynamique de HAUTON et coll., qui respecte strictement un système cohérent d'unités, notamment le système d'unités C.G.S. et l'unité chimique masse/volume $mol.cm^{-3}$ [HAUTON, MARTIGNE, DOMINGO et LAFONT, (1987), BIOCHIMIE 69, pages 547-549 et HAUTON et LAFONT, BIOCHIMIE (1987) 69, pages 177-204]. Cette méthode de calcul permet de définir dans le cas des liposomes multilamellaires, le volume de la phase interfaciale en bicouche et le volume de la phase polaire aqueuse encapsulée, et dans le cas des vésicules unilamellaires, de définir, en plus, le rayon moyen de celles-ci.

Différentes méthodes sont utilisées afin d'obtenir les vésicules multilamellaires ou unilamellaires. L'agitation mécanique de phospholipides hydratés produit ainsi des vésicules multilamellaires (MLV) (OLSON et coll., Biochem., Biophys., Acta 557, 9 - 23) qui, par sonication ou par extrusion à l'aide d'une presse de FRENCH (BARENHOLTZ et coll. (1979), FEBS Lett. 99, 210214 et HAMILTON et coll. (1980), J. Lip. Res. 21, 981 - 992) peuvent être transformés en petits liposomes unilamellaires (SUV); cette catégorie de liposomes peut également être préparée par la technique d'injection d'éthanol (BATZI et KORN (1973), Biochem. Biophys., Acta 298, 1015 1019) ou par l'élimination de détergent par dialyse (KAGAWA et coll., (1971), J. Biol. Chem. 246, 5477 5487, HILSMANN et coll. (1978), Biochem. Biophys., Acta 512, 147 155, ZUMBUEHL et coll. (1981), Biochem. Biophys., Acta 640, 252 262, RHODEN et coll. (1979), Biochemistry 18, 4173 4176) ou par filtration sur gel (BRUNNER et coll. (1976), Biochem. Biophys., Acta 455, 322 - 331 et ENOCH et coll. (1979), Proc. Natl. Acad. Sci. USA 76, 145 - 149) ; les grands liposomes unilamellaires (LUV) peuvent être préparés soit par évaporation en phase reverse (SZAKA et coll.(1978), Proc. Natl. Acad. Sci. USA 75, 41944198 et SZAKA et coll. (1980), Biochem. Biophys., Acta 601, 559 - 571) ou par vaporisation d'éther (DEANER et coll. (1976), Biochem. Biophys., Acta 443, 629 634).

Afin de sélectionner des populations de liposomes SUV de grandes tailles homogènes, on procède soit par ultracentrifugation (WATTS et coll. (1978), Biochemistry 17, 1792-1801), soit par filtration sur gel (HUANG (1969), Biochemistry 9, 344 -352). Pour obtenir des populations homogènes de liposomes MLV, LUV ou SUV, on peut utiliser la technique de filtration sous pression d'azote à travers des membranes de polycarbonate calibrées (OLSON et coll. (1979), Biochem. Biophys., Acta 557, 9 - 23 et SZAKA et coll. (1980), Biochem. Biophys., Acta 601, 559 571).

Toutes ces techniques permettent d'obtenir des préparations de liposomes qui peuvent retenir encapsulés des composés hydrosolubles, dont la quantité dépend du volume de l'espace aqueux encapsulé par mole de lipide et du pourcentage de composé ajouté.

La stabilité des liposomes dépend de nombreux paramètres, tels que la stabilité chimique des lipides, le maintien de la structure des vésicules, la formation d'agrégats de vésicules et/ou de produits de fusion, et la rétention des contenus encapsules dans différentes conditions.

Or, un obstacle important à l'utilisation des liposomes, tels que préparés par les techniques ci-dessus, est constitue par une instabilité de ces particules en fonction du temps, se traduisant par des agrégations, des fusions, des ruptures et des dégradations enzymatiques souvent d'origine bactérienne.

Il a été proposé dans la Demande de Brevet Européen N° 0 162 724, afin d'éviter les problèmes d'agrégation et de fusion des liposomes, de stocker des liposomes dans une matrice de gel polymérique constituée par exemple de polysaccharides et de polypeptides. Dans ces conditions, la taille et les propriétés des liposo-

EP 0 393 049 B1

mes ne sont pas modifiées pendant le stockage, tant que le gel demeure à l'état solide. Lorsqu'on expose le gel à la température ambiante, ou à une température supérieure, le gel va fondre, les liposomes se retrouveront sous forme de suspension dans un milieu aqueux et pourront être ainsi utilisés ultérieurement de la même manière que des liposomes fraîchement préparés.

Il a également été proposé dans la Demande de Brevet Européen n° 0 277 776 (état de la Technique selon l'article 54, paragraphe 3 CBE) de préparer des liposomes à noyau central gélifié en 4 étapes majeures.

De tels liposomes présentent l'inconvénient d'être difficiles à obtenir et de fournir par une succession de procédures des microsphérules aptes à être encapsulées dans des liposomes d'un diamètre important compris entre 5 000 et 60 000 Å.

La présente invention s'est en conséquence donné pour but de pourvoir à un autre moyen de préparer des liposomes que celui proposé par la Demande de Brevet Européen N° 0 162 724, ce moyen permettant d'obtenir des liposomes stables dans le temps avec d'excellents rendements et dans des conditions économiques intéressantes.

La nouvelle classification des lipides proposée par le Professeur HAUTON et coll. basée sur les coefficients de partage $K_D$ entre une phase polaire aqueuse et une phase interfaciale en monocouche ou en bicouche et $K_C$ entre une phase interfaciale et une phase hydrophobe ou apolaire sera utilisée (HAUTON et LAFONT, (1987), BIOCHIMIE, 69, 177-204). Les concentrations en mol.cm$^{-3}$ d'une espèce moléculaire X par cm$^3$ d'un système multiphase sont exprimées par [X] dans le système multiphase, par $[X_E]$ dans la phase polaire aqueuse, par $X_D$ dans la phase interfaciale en monocouche ou en bicouche, par $X_C$ dans la phase apolaire. Les coefficients de partage $K_D$ et $K_C$ expriment respectivement les rapports $X_D/[X_E]$ et $X_D/X_C$.

Selon cette classification quantitative des lipides, on répertorie le classes suivantes :

$$- \text{ Classe 1} \quad \begin{cases} K_C = 0 \\ K_D/K_C = \infty \end{cases}$$

$$- \text{ Classe 2} \quad \begin{cases} K_C < 1 \\ K_D/K_C = \infty \end{cases}$$

$$- \text{ Classe 3} \quad \begin{cases} K_C > 1 \\ K_D/K_C = \infty \end{cases}$$

$$- \text{ Classe 4} \quad \begin{cases} K_C \text{ et } K_D = \infty \\ K_D/K_C = 0 \end{cases}$$

$$- \text{ Classe 5} \quad \begin{cases} 5A \quad \longleftarrow \quad K_D/K_C = 0 \\ 5B \quad \longleftarrow \quad K_D \text{ mesurable} \end{cases}$$

$$- \text{ Classe 6} \quad \begin{cases} K_D/K_C = 0 \\ K_D = 0 \end{cases}$$

3

La classe 4 représente les espèces moléculaires clés des phases interfaciales en monocouche ou en bicouche dans lesquelles peuvent être incorporées également des espèces moléculaires de la classe 5, 3 ou 2. Les lipides de la classe 5 sont des molécules amphiphiles solubles formant des micelles. Les espèces moléculaires de la classe 6 ne sont pas des lipides, mais des molécules hydrosolubles.

La présente invention a pour objet des liposomes du type comportant au moins une couche lipidique externe et au moins une phase polaire aqueuse interne gélifiable, caractérisés en ce que :

a) les liposomes sont préparés en utilisant comme phase polaire aqueuse une solution aqueuse contenant une substance gélifiante à une concentration appropriée, à une température supérieure au point de fusion de ladite substance,

b) les liposomes formés, en suspension dans de la phase polaire aqueuse contenant de la substance gélifiante, sont séparés de cette dernière par dilution de celle-ci pour diminuer la concentration de la substance gélifiante et la rendre ingélifiable, puis

c) les liposomes ainsi préparés sont refroidis à une température inférieure au point de fusion de la substance gélifiante pour fournir des liposomes comportant une phase polaire aqueuse interne gélifiée stable, lesquels liposomes sont en suspension dans une phase polaire aqueuse à l'état liquide.

Les liposomes ainsi gélifiés ne fusionnent pas entre eux lors des collisions, et la vitesses moyenne des molécules est fortement diminuée au sein de la phase polaire gélifiée, encapsulée dans les liposomes.

Selon une disposition avantageuse de la présente invention, la substance gélifiée est un gel polymérique de polysaccharides et de polypeptides.

Selon une autre disposition avantageuse de la présente invention, la substance gélifiée est de la gélatine.

Selon encore une autre disposition avantageuse de la présente invention, la substance gélifiée est l'agarose.

Selon un mode de réalisation avantageux de la présente invention, la substance gélifiée est choisie parmi les substances gélifiantes dont le point de fusion est supérieur à la température corporelle, pour former des liposomes gélifiés stables dont la phase polaire aqueuse gélifiée est apte à être dégradée lentement dans l'organisme et à libérer de façon retardéé la(es) substance(s) incorporée(s) dans ladite phase.

Selon un autre mode de réalisation avantageux des liposomes objet de la présente invention, la phase polaire aqueuse gélifiée contient du saccharose.

Selon un mode de réalisation avantageux des liposomes conformes à la présente invention, ceux-ci sont constitués par une phase interfaciale en bicouche dans le cas de liposomes unilamellaires ou une pluralité de phases interfaciales en bicouche superposées concentriquement, dans le cas de liposomes multilamellaires, et par une phase polaire aqueuse interne encapsulée gélifiée.

Selon une disposition avantageuse de ce mode de réalisation, la ou les phase(s) interfaciale(s) contien(nen)t des produits liposolubles des classes 2 à 5 et/ou la phase polaire aqueuse interne encapsulée gélifiée contient des produits hydrosolubles des classes 5 et 6, les liposomes conformes à l'invention servant ainsi de vecteurs d'espèces moléculaires des classes 2 à 6, notamment de produits à action pharmacologique.

La présente invention a également pour objet un procédé de préparation de liposomes par formation d'au moins une phase interfaciale en bicouche, constituée par des lipides de la classe 4 éventuellement associés à des lipides de la classe 2 et de la classe 3 et/ou des lipides de la classe 5, entourant au moins une phase polaire aqueuse interne, lequel procédé est caractérisé en ce que les liposomes sont préparés en utilisant comme phase polaire aqueuse une solution aqueuse contenant une substance gélifiante à une concentration appropriée, à une température supérieure au point de fusion de ladite substance, en ce que les liposomes formés, en suspension dans la phase polaire aqueuse contenant de la substance gélifiante, sont séparés de cette dernière par dilution de celle-ci pour diminuer la concentration de la substance gélifiante et la rendre ingélifiable, et en ce que les liposomes ainsi séparés sont refroidis à une température inférieure au point de fusion de la substance gélifiante pour fournir des liposomes comportant une phase polaire aqueuse interne gélifiée stable, lesquels liposomes sont en suspension dans une phase polaire aqueuse à l'état liquide.

Selon encore un autre mode de mise en oeuvre avantageux du procédé conforme à la présente invention, la concentration de la substance gélifiante est comprise entre 5 et 10 % en poids par rapport au volume de la phase aqueuse.

Selon un mode de mise en oeuvre avantageux du procédé conforme à la présente invention, on ajuste la densité de la solution polaire aqueuse contenant la substance gélifiante en fonction de la densité que l'on cherche à conférer aux liposomes, à l'aide d'agents de densification appropriés.

Selon une disposition avantageuse de ce mode de mise en oeuvre, la densité est ajustée par adjonction de saccharose à une concentration appropriée, à ladite solution aqueuse.

Des familles de liposomes de densités différentes peuvent ainsi être séparées entre elles lorsqu'elles sont mises en suspension dans une phase aqueuse de densité intermédiaire.

Selon un mode de mise en oeuvre avantageux du procédé conforme à la présente invention, ladite substance gélifiante est choisie dans le groupe qui comprend notamment les gels polymériques de polysaccharides et de polypeptides, la gélatine et l'agarose.

Selon un autre mode de mise en oeuvre avantageux du procédé conforme à la présente invention, les liposomes sont préparés en formant des micelles mixtes de lipides appropriés dans une phase polaire aqueuse contenant 5 à 10 % en poids environ de substance gélifiante, à une température de l'ordre de 45°C environ, supérieure au point de fusion de la substance gélifiante, puis en diluant ou en dialysant les micelles obtenues pour éliminer les lipides amphiphiles solubles des classes 5A et 5B, tout en maintenant la température à 45°C environ, pour transformer lesdites micelles en liposomes unilamellaires, en diluant ensuite la phase polaire aqueuse contenant les liposomes en suspension, pour diminuer sa concentration en substance gélifiante et la rendre ingélifiable, puis en abaissant la température de la suspension de liposomes au-dessous du point de fusion de la substance gélifiante pour gélifier la phase aqueuse interne encapsulée desdits liposomes.

Selon une disposition avantageuse de ce mode de mise en oeuvre, les micelles mixtes sont constituées par des mélanges de sels biliaires et/ou de lécithines et/ou de cholestérol.

Selon une disposition avantageuse de ce mode de mise en oeuvre, la phase polaire aqueuse mise en oeuvre pour la préparation des micelles contient en outre du saccharose à une concentration appropriée, pouvant aller jusqu'à 15 %, et la dilution ou la dialyse subséquente est réalisée respectivement par ou contre une solution aqueuse sans lipides et contenant la même concentration en saccharose que la phase polaire aqueuse susdite, les liposomes obtenus présentant une densité fonction de la concentration en saccharose mise en oeuvre.

Selon une modalité avantageuse de cette disposition, la phase aqueuse contenant des lipides comprend des mélanges de lécithines contenant des sels biliaires et éventuellement du cholestérol.

Selon une autre disposition avantageuse de ce mode de mise en oeuvre, la dilution de la phase polaire aqueuse contenant les liposomes en suspension est réalisée à l'aide d'une solution aqueuse dont la densité est ajustée, de préférence à l'aide de saccharose, pour être différente de celle des liposomes et faciliter de ce fait la séparation de ces derniers, les liposomes d'une densité supérieure sédimentant et les liposomes d'une densité inférieure surnageant.

Selon un mode de mise en oeuvre avantageux du procédé conforme à la présente invention, des liposomes multilamellaires sont préparés en substituant au processus de dilution ou de dialyse mis en oeuvre pour la préparation de liposomes unilamellaires, à partir de micelles mixtes, un processus de sonication, en l'absence de lipides amphiphiles solubles de la classe 5A ou 5B, suivi d'une filtration sur gel permettant de séparer les vésicules multilamellaires des petits et grands liposomes unilamellaires au-dessus de la température de fusion de la substance gélifiante utilisée.

Conformément à l'invention, la phase polaire aqueuse contenant une substance gélifiante et éventuellement un agent d'ajustement de la densité, contient en outre des produits hydrosolubles des classes 5 et 6 à visées pharmacologiques et/ou la phase lipidique contient des produits liposolubles des classes 2 à 5 à visées pharmacologiques.

L'invention sera mieux comprise à l'aide du complément de description qui va suivre, qui se réfère à des exemples de mise en oeuvre du procédé conforme à la présente invention.

Il doit être bien entendu toutefois, que ces exemples sont donnés uniquement à titre d'illustration de l'objet de l'invention, dont ils ne constituent en aucune manière une limitation.

EXEMPLE 1

Préparation de petits liposomes unilamellaires (SUV)

Pour un volume donné d'une phase polaire aqueuse contenant de 5 à 15 % en poids de gélatine, on prépare à une température de 45°C des micelles mixtes sels biliaires-lécithines. Ces micelles mixtes sont ensuite dialysées à 45°C afin d'abaisser la concentration des sels biliaires de la classe 5B, ce qui a pour résultat de transformer lesdites micelles en petits liposomes unilamellaires. Il est procédé à une dilution diminuant la concentration en gélatine de la phase polaire aqueuse contenant la suspension de liposomes, ce qui permet de rendre ladite phase polaire aqueuse ingélifiable. L'abaissement ultérieur de la température au-dessous du point de fusion de la gélatine sélectionnée transforme la phase aqueuse interne des vésicules unilamellaires isolées, en un gel.

La dialyse peut être remplacée d'emblée par une dilution par une solution aqueuse appropriée afin que la concentration des sels biliaires de la classe 5B soit suffisamment abaissée pour transformer les micelles mixtes en petites vésicules unilamellaires.

EXEMPLE 2

Préparation de petits liposomes unilamellaires (SUV) avec utilisation de saccharose.

On suit la procédure décrite dans l'Exemple précédent, mais l'on ajoute, en outre, du saccharose à la phase polaire aqueuse à une concentration pouvant aller jusqu'à 15 % ; on effectue la dialyse contre une solution contenant une même concentration en saccharose, ce qui permet d'obtenir des liposomes dont la phase aqueuse gélifiée a une densité qui est fonction de la concentration de saccharose utilisée. Suivant la densité de la solution aqueuse utilisée lors de la dilution finale, les liposomes sédimentent ou surnagent. En effet, si la dilution finale est faite avec une solution aqueuse de densité moindre que la solution aqueuse gélifiante mise en oeuvre, les liposomes sédimentent, alors que si la solution finale est faite avec une solution aqueuse de densité supérieure à la solution aqueuse gélifiante mise en oeuvre, les liposomes surnagent.

EXEMPLE 3

Préparation de grands liposomes unilamellaires (LUV)

Les LUV sont préparés selon des techniques connues en elles-mêmes décrites dans la Littérature dont il est fait mention dans le préambule de la présente Demande de Brevet, à ceci près que ces techniques sont modifiées conformément à la présente invention par l'addition d'une substance gélifiante à une concentration appropriée et en opérant au-dessus du point de fusion de cette substance.

EXEMPLE 4

Préparation de vésicules multilamellaires (MLV)

Les MLV sont préparées selon des techniques connues en elles-mêmes décrites dans la Littérature citée plus haut, à ceci près que ces techniques sont modifiées conformément à la présente invention par l'introduction d'une substance gélifiante à une concentration appropriée et par le maintien de la température de préparation au-dessus du point de fusion de ladite substance.

EXEMPLE 5

Application des liposomes conformes à l'invention comme vecteurs de produits à action pharmacologique

En choisissant une substance gélifiante dont le point de fusion est supérieur à la température corporelle, par exemple en utilisant de la gélatine modifiée chimiquement pour porter son point de fusion, qui est normalement de 37°C, à 45°C, et en ajoutant en outre à la phase aqueuse, un agent thérapeutique, on prépare des liposomes gélifiés stables dont la phase polaire aqueuse gélifiée sera lentement dégradée par l'organisme, ce qui se traduit par une libération progressive, programmée des agents thérapeutiques incorporés dans la phase aqueuse gélifiée, et confère aux médicaments en question une action retard qui peut s'étendre sur des durées prolongées.

EXEMPLE 6

Application des liposomes conformes à l'invention dans des processus de diagnostic.

Il est possible d'utiliser les liposomes stables conformes à la présente invention dans des processus de diagnostic, tel que notamment la néphélométrie laser dans les techniques d'immunoprécipitation en milieu liquide, dans lesquelles les particules de latex actuellement utilisées pourront être avantageusement remplacées par des particules plus petites constituées par des SUV gélifiés stables.

Des ligands divers peuvent être associés à la phase interfaciale en bicouche pour favoriser la captation des liposomes par des sites de réception donnés.

**Revendications**

1. Liposomes du type comportant au moins une couche lipidique externe et au moins une phase polaire aqueuse interne gélifiable, caractérisés en ce que :

a) les liposomes sont préparés en utilisant comme phase polaire aqueuse une solution aqueuse contenant une substance gélifiante à une concentration appropriée, à une température supérieure au point de fusion de ladite substance,

b) les liposomes formés, en suspension dans de la phase polaire aqueuse contenant de la substance gélifiante, sont séparés de cette dernière par dilution de celle-ci pour diminuer la concentration de la substance gélifiante et la rendre ingélifiable, puis

c) les liposomes ainsi préparés sont refroidis à une température inférieure au point de fusion de la substance gélifiante pour fournir des liposomes comportant une phase polaire aqueuse interne gélifiée stable, lesquels liposomes sont en suspension dans une phase polaire aqueuse à l'état liquide.

2.  Liposomes selon la revendication 1, caractérisés en ce que la substance gélifiante est un gel polymérique de polysaccharides et de polypeptides.

3.  Liposomes selon la revendication 1, caractérisés en ce que la substance gélifiante est de la gélatine.

4.  Liposomes selon la revendication 1, caractérisés en ce que la substance gélifiante est l'agarose.

5.  Liposomes selon la revendication 1, caractérisés en ce que la substance gélifiante est choisie parmi les substances gélifiantes dont le point de fusion est supérieur à la température corporelle, pour former des liposomes gélifiés stables dont la phase polaire aqueuse gélifiée est apte à être dégradée lentement dans l'organisme et à libérer de façon retardée la(les) substances(s) incorporée(s) dans ladite phase.

6.  Liposomes selon l'une quelconque des revendications 1 à 5, caractérisés en ce que la phase polaire aqueuse gélifiée contient du saccharose.

7.  Liposomes selon l'une quelconque des revendications 1 à 6, caractérisés en ce que ceux-ci sont constitués par une phase interfaciale en bicouche dans le cas de liposomes unilamellaires ou une pluralité de phases interfaciales en bicouche superposées concentriquement, dans le cas de liposomes multilamellaires, et par une phase polaire aqueuse interne encapsulée gélifiée.

8.  Liposomes selon la revendication 7, caractérisés en ce que la ou les phase(s) interfaciale(s) contien(nen)t des produits liposolubles de classe 2 (notamment triglycérides à longue chaînes, esters de cholestérol), de classe 3 (notamment cholestérol, acides gras à longue chaîne non ionisés), de classe 4 (notamment phospholipides, monoglycérides à longue chaîne), de classe 5 (notamment sels biliaires, dérivés de l'acide fusidique) et/ou la phase polaire aqueuse interne encapsulée gélifiée contient des produits hydrosolubles des classes 5 (notamment lysophospholipide) et 6 (molécules hydrophiles), les liposomes servant ainsi de vecteurs d'espèces moléculaires des classes 2 à 6, notamment de produits à action pharmacologique.

9.  Procédé de préparation de liposomes par formation d'au moins une phase interfaciale en bicouche, constituée par des lipides de la classe 4, éventuellement associés à des lipides de la classe 2 et de la classe 3 et/ou des lipides de la classe 5 entourant au moins une phase polaire aqueuse interne, lequel procédé est caractérisé en ce que :

a) les liposomes sont préparés en utilisant comme phase polaire aqueuse une solution aqueuse contenant une substance gélifiante à une concentration appropriée, à une température supérieure au point de fusion de ladite substance,

b) les liposomes formés, en suspension dans de la phase polaire aqueuse contenant de la substance gélifiante, sont séparés de cette dernière par dilution de celle-ci pour diminuer la concentration de la substance gélifiante et la rendre ingélifiable, puis

c) les liposomes ainsi préparés sont refroidis à une température inférieure au point de fusion de la substance gélifiante pour fournir des liposomes comportant une phase polaire aqueuse interne gélifiée stable, lesquels liposomes sont en suspension dans une phase polaire aqueuse à l'état liquide.

10.  Procédé selon la revendication 9, caractérisé en ce que la concentration de la substance gélifiante est comprise entre 5 et 10 % en poids par rapport au volume de la phase aqueuse.

11.  Procédé selon l'une quelconque des revendications 9 et 10, caractérisé en ce qu'on ajuste la densité de la solution polaire aqueuse contenant la substance gélifiante, en fonction de la densité que l'on cherche à conférer aux liposomes, à l'aide d'agents de densification appropriés.

**12.** Procédé selon la revendication 11, caractérisé en ce que la densité est ajustée par adjonction de saccharose à une concentration appropriée, à ladite solution aqueuse.

**13.** Procédé selon l'une quelconque des revendications 9 à 12, caractérisé en ce que ladite substance gélifiante est choisie dans le groupe qui comprend notamment les gels polymériques de polysaccharides et de polypeptides, la gélatine et l'agarose.

**14.** Procédé selon l'une quelconque des revendications 9 à 13, caractérisé en ce que les liposomes sont préparés en formant des micelles mixtes de lipides appropriés dans une phase polaire aqueuse contenant 5 à 10 % en poids environ de substance gélifiante, à une température de l'ordre de 45°C environ, supérieure au point de fusion de la substance gélifiante, puis en diluant ou dialysant les micelles obtenues, pour éliminer les lipides amphiphiles solubles des classes 5A et 5B, tout en maintenant la température à 45°C environ, pour transformer lesdites micelles en petits liposomes unilamellaires, en diluant ensuite la phase polaire aqueuse contenant les liposomes en suspension, pour diminuer sa concentration en substance gélifiante et la rendre ingélifiable, puis en abaissant la température de la suspension de liposomes au-dessous du point de fusion de la substance gélifiante pour gélifier la phase aqueuse interne encapsulée desdits liposomes.

**15.** Procédé selon la revendication 14, caractérisé en ce que les micelles sont constituées par des mélanges de sels biliaires et/ou lécithines et/ou de cholestérol.

**16.** Procédé selon la revendication 14, caractérisé en ce que la phase polaire aqueuse mise en oeuvre pour la préparation des micelles contient en outre du saccharose à une concentration appropriée, pouvant atteindre 15 % en poids, et la dilution ou la dialyse subséquente est réalisée par ou contre une solution aqueuse sans lipides et contenant la même concentration en saccharose que la phase polaire aqueuse susdite, les liposomes obtenus présentant une densité fonction de la concentration en saccharose mise en oeuvre.

**17.** Procédé selon la revendication 16, caractérisé en ce que la phase aqueuse lipidique comprend des mélanges de lécithines et de sels biliaires et éventuellement du cholestérol.

**18.** Procédé selon la revendication 14, caractérisé en ce que la dilution de la phase polaire aqueuse contenant les liposomes en suspension est réalisée à l'aide d'une solution aqueuse dont la densité est ajustée, de préférence à l'aide de saccharose, pour être différente de celle des liposomes et faciliter de ce fait la séparation de ces derniers.

**19.** Procédé selon l'une quelconque des revendications 9 à 13, caractérisé en ce que les liposomes multilamellaires sont préparés en substituant au processus de dilution ou de dialyse mis en oeuvre pour la préparation de liposomes unilamellaires à partir de micelles mixtes, un processus de sonication, en l'absence de lipides amphiphiles solubles de la classe 5A ou 5B, suivi d'une filtration sur gel permettant de séparer les vésicules multilamellaires des petits et grands liposomes unilamellaires, au-dessus de la température de fusion de la substance gélifiante utilisée.

**20.** Procédé selon l'une quelconque des revendications 9 à 19, caractérisé en ce que la phase polaire aqueuse contenant une substance gélifiante et éventuellement un agent d'ajustement de la densité, contient en outre des produits hydrosolubles des classes 5 et 6 à visées pharmacologiques et/ou la phase lipidique contient des produits liposolubles des classes 2 à 5 à visées pharmacologiques.

**Patentansprüche**

**1.** Liposomen des Typs, der mindestens eine externe Lipidschicht und mindestens eine wäßrige, polare, innere, gelbildende Phase trägt, dadurch **gekennzeichnet,** daß:

a) die Liposomen unter Verwendung einer wäßrigen Lösung, die eine gelbildende Substanz in einer geeigneten Konzentration bei einer Temperatur über dem Schmelzpunkt der Substanz enthält, als polare, wäßrige Phase hergestellt werden,

b) die gebildeten Liposomen, die in Suspension in der polaren, wäßrigen Phase, die die gelbildende Substanz enthält, vorliegen, von der zuletzt genannten durch deren Verdünnung abgetrennt werden, um die Konzentration der gelbildenden Substanz zu verringern und sie nicht gelierbar zu machen, dann

c) die so hergestellten Liposomen bei einer Temperatur unter dem Schmelzpunkt der gelbildenden Substanz eingefroren werden, um Liposomen zu erzeugen, die eine stabile, gelierte, innere, wäßrige, polare Phase tragen, wobei die Liposomen in Suspension in einer wäßrigen, polaren Phase in flüssigem Zustand vorliegen.

2. Liposomen nach Anspruch 1, dadurch **gekennzeichnet,** daß die gelbildende Substanz ein polymeres Polysaccharid- und Polypeptidgel ist.

3. Liposomen nach Anspruch 1, dadurch **gekennzeichnet**, daß die gelbildende Substanz Gelatine ist.

4. Liposomen nach Anspruch 1, dadurch **gekennzeichnet,** daß die gelbildende Substanz Agarose ist.

5. Liposomen nach Anspruch 1, dadurch **gekennzeichnet,** daß die gelbildende Substanz unter gelbildenden Substanzen, deren Schmelzpunkt über der Körpertemperatur liegt, ausgewählt ist, um stabile, gelierte Liposomen herzustellen, deren gelierte, wäßrige, polare Phase geeignet ist, im Organismus langsam abgebaut zu werden und verzögert die in diese Phase eingearbeitete Substanz bzw. Substanzen freizusetzen.

6. Liposomen nach einem der Ansprüche 1 bis 5, dadurch **gekennzeichnet,** daß die gelierte, wäßrige, polare Phase Saccharose enthält.

7. Liposomen nach einem der Ansprüche 1 bis 6, dadurch **gekennzeichnet,** daß sie aus einer doppelschichtigen Zwischenphase im Falle von unilamellären Liposomen oder einer Vielzahl von zweischichtigen Zwischenphasen, die konzentrisch übereinander gelagert sind, im Falle von multilamellären Liposomen und einer gelierten, verkapselten, inneren, wäßrigen, polaren Phase bestehen.

8. Liposomen nach Anspruch 7, dadurch **gekennzeichnet,** daß die Zwischenphase bzw. Zwischenphasen unlösliche Produkte der Klasse 2 (insbesondere langkettige Triglyceride, Cholesterinester), der Klasse 3 (insbesondere Cholesterin, langkettige nichtionisierte Fettsäuren}, der Klasse 4 (insbesondere Phospholipide, langkettige Monoglyceride), der Klasse 5 (insbesondere Gallensalze, Derivate der Fusidinsäure) enthält bzw. enthalten und/oder die gelierte, verkapselte, innere, wäßrige, polare Phase wasserlösliche Produkte der Klassen 5 (insbesondere Lysophospholipide) und 6 (hydrophile Moleküle) enthält, wobei die Liposomen so als Vektoren der Molekülspecies der Klassen 2 bis 6, insbesondere der Produkte mit pharmakologischer Wirkung, dienen.

9. Verfahren zur Herstellung von Liposomen durch Bildung mindestens einer doppelschichtigen Zwischenphase aus Lipiden der Klasse 4, gegebenenfalls assoziiert mit Lipiden der Klasse 2 und der Klasse 3 und-/oder Lipiden der Klasse 5, die mindestens eine innere, wäßrige, polare Phase umgeben, dadurch **gekennzeichnet, daß:**
a) die Liposomen unter Verwendung einer wäßrigen Lösung, die eine gelbildende Substanz in einer geeigneten Konzentration bei einer Temperatur über dem Schmelzpunkt der Substanz enthält, als polare, wäßrige Phase hergestellt werden,
b) die gebildeten Liposomen, die in Suspension in der polaren, wäßrigen Phase, die die gelbildende Substanz enthält, vorliegen, von der zuletzt genannten durch deren Verdünnung abgetrennt werden, um die Konzentration der gelbildenden Substanz zu verringern und sie nicht gelierbar zu machen, dann
c) die so hergestellten Liposomen bei einer Temperatur unter dem Schmelzpunkt der gelbildenden Substanz eingefroren werden, um Liposomen zu erzeugen, die eine stabile, gelierte, innere, wäßrige, polare Phase tragen, wobei die Liposomen in Suspension in einer wäßrigen, polaren Phase in flüssigem Zustand vorliegen.

10. Verfahren nach Anspruch 9, dadurch **gekennzeichnet,** daß die Konzentration der gelbildenden Substanz zwischen 5 und 10 Gew.-%, bezogen auf das Volumen der wäßrigen Phase, liegt.

11. Verfahren nach einem der Ansprüche 9 und 10, dadurch **gekennzeichnet**, daß man die Dichte der wäßrigen, polaren Lösung, die die gelbildende Substanz enthält, als Funktion der Dichte, die man den Liposomen verleihen möchte, mit Hilfe von geeigneten Mitteln zur Erhöhung der Dichte einstellt.

12. Verfahren nach Anspruch 11, dadurch **gekennzeichnet,** daß die Dichte durch Zugabe von Saccharose in einer geeigneten Konzentration zu der wäßrigen Lösung eingestellt wird.

**13.** Verfahren nach einem der Ansprüche 9 bis 12, dadurch **gekennzeichnet,** daß die gelbildende Substanz aus der Gruppe, umfassend insbesondere polymere Polysaccharid- und Polypeptidgele, Gelatine und Agarose, ausgewählt wird.

**14.** Verfahren nach einem der Ansprüche 9 bis 13, dadurch **gekennzeichnet,** daß die Liposomen durch Bildung von vermischten Micellen aus geeigneten Lipiden in einer wäßrigen, polaren Phase, die etwa 5 bis 10 Gew.-% der gelbildenden Substanz enthält, bei einer Temperatur im Bereich von etwa 45°C, über dem Schmelzpunkt der gelbildenden Substanz, anschließendes Verdünnen oder Dialysieren der erhaltenen Micellen, um die löslichen, amphiphilen Lipide der Klassen 5A und 5B zu eliminieren, wobei das Ganze bei einer Temperatur von etwa 45°C gehalten wird, um die Micellen in kleine, unilamelläre Liposomen zu überführen, durch anschließendes Verdünnen der wäßrigen, polaren Phase, die die Liposomen in Suspension enthält, um deren Konzentration an gelbildender Substanz zu verringern und sie nichtgelierbar zu machen, durch anschließendes Absenken der Temperatur der Liposomensuspension unter den Schmelzpunkt der gelbildenden Substanz, um die wäßrige, innere Phase, die von den Liposomen verkapselt ist, zu gelieren, hergestellt werden.

**15.** Verfahren nach Anspruch 14, dadurch **gekennzeichnet,** daß die Micellen aus Gemischen aus Gallensalzen und/oder Lecithinen und/oder Cholesterin bestehen.

**16.** Verfahren nach Anspruch 14, dadurch **gekennzeichnet,** daß die wäßrige, polare Phase, die zur Herstellung der Micellen verwendet wird, unter anderem Saccharose in einer geeigneten Konzentration, um 15 Gew.-% zu erhalten, enthält und die anschließende Verdünnung oder Dialyse in oder gegen eine wäßrige Lösung ohne Lipide, die die gleiche Saccharosekonzentration wie die obige wäßrige, polare Phase enthält, durchgeführt wird, wobei die erhaltenen Liposomen eine Dichte als Funktion der eingesetzten Saccharosekonzentration besitzen.

**17.** Verfahren nach Anspruch 16, dadurch **gekennzeichnet,** daß die wäßrige Lipidphase Gemische aus Lecithinen und Gallensalzen und gegebenenfalls Cholesterin enthalten.

**18.** Verfahren nach Anspruch 14, dadurch **gekennzeichnet,** daß die Verdünnung der wäßrigen, polaren Phase, die Liposomen in Suspension enthält, mit Hilfe einer wäßrigen Lösung, deren Dichte bevorzugt mit Hilfe von Saccharose so eingestellt wird, daß sie sich von der der Liposomen unterscheidet und dadurch die Abtrennung der letzteren erleichtert, durchgeführt wird.

**19.** Verfahren nach einem der Ansprüche 9 bis 13, dadurch **gekennzeichnet,** daß die multilamellären Liposomen dadurch hergestellt werden, daß man den Verdünnungs- oder Dialyseprozeß, der zur Herstellung unilamellärer Liposomen aus vermischten Micellen verwendet wird, durch ein Ultraschallverfahren in Abwesenheit von löslichen, amphiphilen Lipiden der Klasse 5A oder 5B und anschließende Filtration über ein Gel, das die Abtrennung der multilamellären Bläschen von kleinen und großen unilamellären Liposomen gestattet, über der Schmelztemperatur der verwendeten gelbildenden Substanz ersetzt.

**20.** Verfahren nach einem der Ansprüche 9 bis 19, dadurch **gekennzeichnet,** daß die wäßrige, polare Phase, die eine gelbildende Substanz und gegebenenfalls ein Mittel zur Einstellung der Dichte enthält, unter anderem wasserlösliche Produkte der Klassen 5 und 6 mit pharmakologischen Wirkungen enthält und/oder die Lipidphase lipidlösliche Produkte der Klassen 2 bis 5 mit pharmakologischen Wirkungen enthält.

## Claims

**1.** Liposomes of the type comprising at least one outer lipid layer and at least one gellable inner aqueous polar phase, characterized in that:

a) the liposomes are prepared by using as the aqueous polar phase an aqueous solution containing a gelling substance at an appropriate concentration, at a temperature above the melting point of said substance,

b) the liposomes formed, in suspension in aqueous polar phase containing gelling substance, are separated from said phase by dilution thereof so as to reduce the concentration of the gelling substance and render it ungellable, and then

c) the liposomes prepared in this way are cooled to a temperature below the melting point of the gelling substance in order to produce liposomes comprising a stable gelled inner aqueous polar phase, which liposomes are in suspension in an aqueous polar phase in the liquid state.

2.  Liposomes according to Claim 1, characterized in that the gelling substance is a polymeric gel of polysaccharides and polypeptides.

3.  Liposomes according to Claim 1, characterized in that the gelling substance is gelatin.

4.  Liposomes according to Claim 1, characterized in that the gelling substance is agarose.

5.  Liposomes according to Claim 1, characterized in that the gelling substance is selected from gelling substances whose melting point is above body temperature, so as to form stable gelled liposomes of which the gelled aqueous polar phase is capable of being degraded slowly in the organism and of effecting the delayed release of the substance(s) incorporated in said phase.

6.  Liposomes according to any one of Claims 1 to 5, characterized in that the gelled aqueous polar phase contains sucrose.

7.  Liposomes according to any one of Claims 1 to 6, characterized in that they consist of one interfacial phase as a bilayer in the case of monolamellar liposomes, or several concentrically superimposed interfacial phases as a bilayer in the case of multilamellar liposomes, and of a gelled encapsulated inner aqueous polar phase.

8.  Liposomes according to Claim 7, characterized in that the interfacial phase (or phases) contains (contain) liposoluble products of class 2 (especially long-chain triglycerides, cholesterol esters), class 3 (especially cholesterol, non-ionized long-chain fatty acids), class 4 (especially phospholipids, long-chain monoglycerides) or class 5 (especially bile salts, fusidic acid derivatives), and/or the gelled encapsulated inner aqueous polar phase contains water-soluble products of class 5 (especially lysophospholipids) and class 6 (hydrophilic molecules), the liposomes thus serving as vectors for molecular species of classes 2 to 6, especially products with a pharmacological action.

9.  A method of preparing liposomes by the formation of at least one interfacial phase as a bilayer, consisting of lipids of class 4, associated with lipids of class 2 and class 3 if appropriate, and/or lipids of class 5, surrounding at least one inner aqueous polar phase, which method is characterized in that:
    a) the liposomes are prepared by using as the aqueous polar phase an aqueous solution containing a gelling substance at an appropriate concentration, at a temperature above the melting point of said substance,
    b) the liposomes formed, in suspension in aqueous polar phase containing gelling substance, are separated from said phase by dilution thereof so as to reduce the concentration of the gelling substance and render it ungellable, and then
    c) the liposomes prepared in this way are cooled to a temperature below the melting point of the gelling substance in order to produce liposomes comprising a stable gelled inner aqueous polar phase, which liposomes are in suspension in an aqueous polar phase in the liquid state.

10. A method according to Claim 9, characterized in that the concentration of the gelling substance is between 5 and 10% by weight, relative to the volume of the aqueous phase.

11. A method according to Claim 9 or Claim 10, characterized in that the density of the aqueous polar solution containing the gelling substance is adjusted with the aid of appropriate densifying agents as a function of the density which it is desired to impart to the liposomes.

12. A method according to Claim 11, characterized in that the density is adjusted by the addition of sucrose at an appropriate concentration to said aqueous solution.

13. A method according to any one of Claims 9 to 12, characterized in that said gelling substance is selected from the group comprising especially polymeric gels of polysaccharides and polypeptides, gelatin and agarose.

14. A method according to any one of Claims 9 to 13, characterized in that the liposomes are prepared by forming mixed micelles of appropriate lipids in an aqueous polar phase containing about 5 to 10% by weight of gelling substance, at a temperature of the order of about 45°C, which is above the melting point of the gelling substance, then by diluting or dialyzing the micelles obtained so as to remove the soluble amphiphilic lipids of classes 5A and 5B, while at the same time holding the temperature at about 45°C, in order to convert said micelles to small monolamellar liposomes, then by diluting the aqueous polar phase containing the liposomes in suspension so as to reduce its concentration of gelling substance and render it ungellable, and then by lowering the temperature of the liposome suspension below the melting point of the gelling substance so as to gel the encapsulated inner aqueous phase of said liposomes.

15. A method according to Claim 14, characterized in that the micelles consist of mixtures of bile salts and/or lecithins and/or cholesterol.

16. A method according to Claim 14, characterized in that the aqueous polar phase used for the preparation of the micelles also contains sucrose at an appropriate concentration, which can be as much as 15% by weight, and the subsequent dilution or dialysis is carried out with or against a lipid-free aqueous solution having the same sucrose concentration as the above-mentioned aqueous polar phase, the density of the liposomes obtained being a function of the sucrose concentration used.

17. A method according to Claim 16, characterized in that the aqueous lipid phase comprises mixtures of lecithins and bile salts and, if appropriate, cholesterol.

18. A method according to Claim 14, characterized in that the dilution of the aqueous polar phase containing the liposomes in suspension is effected with an aqueous solution whose density is adjusted, preferably with sucrose, so as to be different from that of the liposomes and hence to facilitate the separation of said liposomes.

19. A method according to any one of Claims 9 to 13, characterized in that the multilamellar liposomes are prepared by replacing the dilution or dialysis process, used to prepare monolamellar liposomes from mixed micelles, with a sonication process, in the absence of soluble amphiphilic lipids of class 5A or 5B, followed by gel filtration making it possible to separate the multilamellar vesicles from the small and large monolamellar liposomes, above the melting point of the gelling substance used.

20. A method according to any one of Claims 9 to 19, characterized in that the aqueous polar phase containing a gelling substance and, if appropriate, a density adjusting agent also contains water-soluble products of classes 5 and 6 for pharmacological purposes, and/or the lipid phase contains liposoluble products of classes 2 to 5 for pharmacological purposes.